# EUROPEAN PATENT APPLICATION

(11) **EP 4 389 019 A1**
(43) Date of publication of application: **26.06.2024**
(21) Application number: 22950901.3
(22) Date of filing: 03.11.2022
(51) Int. Cl.: A61B 17/12, H01F 7/14, A61B 17/00

(54) **ROTARY SELF-LOCKING MECHANISM, REVERSIBLE SELF-LOCKING CLAMPING APPARATUS, AND CLAMPING METHOD**

(30) Priority: 12.07.2022 CN 202210815021
(71) Applicant: Jilin University, Changchun, Jilin 130012 (CN)
(72) Inventor: CUI, Shusen, Changchun, Jilin 130033 (CN); LIU, Xilin, Changchun, Jilin 130033 (CN)
(74) Representative: Zaboliene, Reda
(86) International application number: PCT/CN2022/129515
(87) International publication number: WO 2024/011793

(57) **Abstract**

A rotary self-locking mechanism, a reversible self-locking clamping apparatus, and a clamping method. The reversible self-locking clamping apparatus comprises a fixing support, a rotating mechanism, a driving locking mechanism, and a clamping mechanism; the driving locking mechanism comprises a magnet and a locking assembly; the magnet responds to an applied external rotating magnetic field to provide an action force used for driving the rotation of the rotating mechanism; the locking assembly responds to the applied external rotating magnetic field to unlock the rotating mechanism; the locking assembly locks the rotating mechanism after the external rotating magnetic field is removed, so that the rotating mechanism cannot rotate; the clamping mechanism responds to the rotation of the rotating mechanism to clamp and compress or release a target object that needs to be clamped and compressed. According to the rotary self-locking mechanism, the reversible self-locking clamping apparatus, and the clamping method, an animal model through which a reversible compression force is applied to the target object can be constructed, such that an experimental result would not be affected by stress stimulation such as an operation.

## Description

### Technical Field

The present disclosure relates to the technical field of biomedical engineering, and in particular to a rotary self-locking mechanism, a reversible self-locking clamping apparatus and a clamping method.

### Background Art

In the scientific research process of acute and chronic ischemia, ischemia-reperfusion model (heart, brain, spinal cord, kidney and intestinal tract), spinal cord nerve compression or peripheral nerve injury, it is necessary to establish an animal model by acute and chronic compression on animal blood vessels or nerves to simulate corresponding conditions, so as to verify the effect of drugs or treatments.

In the traditional modeling method, a compression model is constructed by surgically ligating around a vessel or nerve in the animal to be compressed with sutures or placing a flexible tube of smaller diameter than the vessel or nerve. In the suture compression method, 4 ligations are performed on selected vessels or nerves by the surgical suture, with an interval of about 1 mm between each ligation. We artificially judge the compression amount, and construct an acute compression model without cutting off the blood supply. In the construction of chronic entrapment model, the surgical suture is replaced by the catgut which is used as a water-swellable material. After absorption of body fluid, the catgut will gradually swell to increase the amount of compression, and the compression rate is affected by the secretion rate of body fluid. Among them, in the hose compression method, a silicone hose with smaller diameter than blood vessel or nerve is surgically implanted at a selected position for constructing acute compression. The chronic constriction model is constructed by stimulating the outer wall of the vessel or nerve with the implanted silicone hose to produce an inflammatory response and cause congestion and swelling, thereby compressing the inner wall when the outer wall is restricted by the hose. The amount of compression will vary depending on the degree of the inflammatory response of the individual immune system to the hose.

Currently existing models for acute or chronic compression are generally constructed with the following deficiencies.
1. The construction of the compression model of vessels or nerves is highly influenced by human factors. Since the traditional acute compression model is usually completed by the procedure and the amount of compression is determined manually by the operator, there is a lack of quantitative indicators. Moreover, the constriction occurs from the time of operation and is accompanied by a part of vascular or nerve injury caused by the surgical operation process. It is impossible to exclude the influence of injury during the operation on the experimental results, thus increasing the unreliability of experimental results.
2. The construction of chronic compression models of blood vessels or nerves is greatly influenced by individual factors. Both the secretion rate of body fluid and the swelling rate of outer wall are different due to individual differences of experimental animals, so that the amount of chronic compression cannot be accurately designed or measured, thus increasing the uncontrollable factors in the experiment.
3. The compression rate of chronic compression model is uncontrollable, which is determined by the individual's body fluid secretion rate or inflammatory response rate. For some special individuals, since the body fluid secretion rate is too fast, it is impossible to construct chronic compression with a sufficiently slow speed, thus limiting the experimental method and object.
4. The chronic compression method is not reversible. Due to the irreversibility of the water-swellable material and the uncontrollable inflammation swelling, all the chronic compression modeling methods can only achieve gradual increase of compression degree, but cannot achieve gradual reduction of compression. Therefore, it cannot simulate the process of compression symptom relief, thus limiting the symptoms targeted by the experiment.

Generally, in order to avoid the influence of human factors and individual differences of animals, it is often necessary to use a large number of experimental animal samples to weaken the deviation of experimental results caused by individual differences, thus consuming a lot of experimental resources and costs. However, there is no reliable technical means to realize the defect that the compression degree is irreversible.

### Summary of the Invention

In one aspect, it is an object of the present disclosure to provide a reversible self-locking clamping apparatus to construct an animal model through which a reversible compression force is applied to the target object, such that an experimental result would not be affected by stress stimulation such as an operation.

In order to achieve the above object, the present disclosure provides a reversible self-locking clamping apparatus, comprising a fixing support configured for fixing the reversible self-locking clamping apparatus inside the body of an experimental animal, a rotating mechanism rotatably mounted on the fixing support, a driving locking mechanism, and a clamping mechanism drivingly connected to the rotating mechanism, wherein the driving locking mechanism comprises a magnet and a locking assembly; the magnet is capable of providing an action force for driving the rotation of the rotating mechanism in response to an applied external rotating magnetic field; the locking assembly is capable of unlocking the rotating mechanism in response to the applied external rotating magnetic field; the locking assembly locks the rotating mechanism after the external rotating magnetic field is removed so that the rotating mechanism cannot rotate; and the clamping mechanism is capable of clamping and compressing or releasing a target object that needs to be clamped in response to the rotation of the rotating mechanism.

In a preferred embodiment, the locking assembly comprises an iron core, a reset element, a lock head and a lock hole, wherein the iron core is capable of driving the lock head away from the lock hole under the action of an external rotating magnetic field to unlock the rotating mechanism; and the reset element drives the iron core into the lock hole to lock the rotating mechanism when the external rotating magnetic field is removed.

In a preferred embodiment, the lock hole is a tapered counterbore.

In a preferred embodiment, the reset element is a spring.

In a preferred embodiment, the fixing support comprises a rotary shaft; the rotating mechanism comprises a turntable which is rotatably mounted on the rotary shaft; and the magnet is mounted on the turntable and located at an outer part of the turntable.

In a preferred embodiment, an outer through-hole and an inner through-hole are disposed on the turntable; the outer through-hole (24) and the inner through-hole are coaxially disposed; the magnet is fixedly mounted in the outer through-hole; the iron core is slidably mounted in the inner through-hole; the lock head is fixed on the iron core; the reset element is disposed between the iron core and the magnet; the lock holes are formed on the rotary shaft; and the lock holes are uniformly distributed around the axis of the rotary shaft.

In a preferred embodiment, the rotating mechanism further comprises a rotary drum rotatably nested on the rotary shaft; the rotary drum is fixedly connected to and coaxially provided with the turntable; and the outer diameter of the rotary drum is less than the outer diameter of the turntable.

In a preferred embodiment, the reversible self-locking clamping apparatus further comprises a housing; the housing is fixed to the fixing support; and the rotating mechanism is disposed inside the housing.

In a preferred embodiment, the clamping mechanism comprises a locking strap, which is capable of being wrapped outside the target object; the housing is provided with a slit; one end of the locking strap is connected to the inside of the housing or the edge of the slit, and the other end of the locking strap passes through the slit and is connected to the rotating mechanism.

In a preferred embodiment, the locking strap is made of a biocompatible resin.

In a preferred embodiment, the fixing support comprises a leg and a rotary shaft fixedly mounted on the leg; and the leg is provided with a pinhole for suture-fixing the leg in vivo of the animal.

The present disclosure differs from the prior art in that after the reversible self-locking clamping apparatus provided by the present disclosure is mounted inside the body of an experimental animal, the locking assembly responds to an external rotating magnetic field when a positive external rotating magnetic field is applied outside the animal body to unlock the rotating mechanism. At this time the rotating mechanism can rotate on the fixing support. Also, the magnet will generate a magnetically induced force under the action of the external rotating magnetic field, and the direction thereof is the direction of the magnetic line of maximum force pointing to the external magnetic field, i.e., generating a force to drive the magnetic pole direction of the magnet to coincide with the external magnetic field, thereby driving the rotating mechanism to rotate. The rotating mechanism drives the clamping mechanism to apply clamping and compression to the target object (such as vascular or neural pressure) while rotating. When the external rotating magnetic field is removed, the locking assembly locks the rotating mechanism, so that the rotating mechanism does not rotate any more. Since the clamping mechanism is drivingly connected with the rotating mechanism, the clamping mechanism also remains stationary. At this time, a specific amount of compression may be continuously applied to the target object. When it is necessary to change the amount of compression on the target object, it is only necessary to apply a rotating magnetic field outside the animal body again, and the compression force applied on the target object by the clamping mechanism can be accurately adjusted by controlling the rotation angle and the number of turns of the rotating magnetic field. For example, when it is desired to reduce the amount of compression to which the target object is subjected, only a counter-rotating magnetic field may be applied. Therefore, the reversible self-locking clamping apparatus provided by the present disclosure may construct an animal model through which a reversible compression force is applied to the target object. Meanwhile, since the reversible self-locking clamping apparatus provided by the present disclosure gradually compresses the target subject by non-contact application of the external rotating magnetic field, an experimental result would not be affected by stress stimulation such as an operation.

It is another object of the present disclosure to provide a rotary self-locking mechanism that can be applied to a reversible self-locking clamping apparatus to construct an animal model through which a reversible compression force is applied to the target object.

In order to achieve the above object, the technical solution of the present disclosure is achieved as follows.

A rotary self-locking mechanism comprises a rotary shaft, a rotating mechanism rotatably mounted on the rotary shaft, and a driving locking mechanism. The driving locking mechanism comprises a magnet and a locking assembly; the magnet is capable of providing an action force for driving the rotation of the rotating mechanism in response to an applied external rotating magnetic field; the locking assembly is capable of unlocking the rotating mechanism in response to the applied external rotating magnetic field; and the locking assembly locks the rotating mechanism after the external rotating magnetic field is removed so that the rotating mechanism cannot rotate.

In a preferred embodiment, the locking assembly comprises an iron core, a reset element, a lock head and a lock hole, wherein the iron core is capable of driving the lock head away from the lock hole under the action of an external rotating magnetic field to unlock the rotating mechanism; and the reset element drives the iron core into the lock hole to lock the rotating mechanism when the external rotating magnetic field is removed.

In a preferred embodiment, the lock hole is a tapered counterbore; and the reset element is a spring.

In a preferred embodiment, the fixing support comprises a rotary shaft; the rotating mechanism comprises a turntable which is rotatably mounted on the rotary shaft; and the magnet is mounted on the turntable and located at an outer part of the turntable.

In a preferred embodiment, an outer through-hole and an inner through-hole are disposed on the turntable; the outer through-hole (24) and the inner through-hole are coaxially disposed; the magnet is fixedly mounted in the outer through-hole; the iron core is slidably mounted in the inner through-hole; the lock head is fixed on the iron core; the reset element is disposed between the iron core and the magnet; the lock holes are formed on the rotary shaft; and the lock holes are uniformly distributed around the axis of the rotary shaft.

The rotary self-locking mechanism has the same technical advantages as the reversible self-locking clamping apparatus described above with respect to the prior art and will not be described in detail here.

In another aspect, the present disclosure also provides a clamping method that enables reversible compression on a target object.

In order to achieve the above object, the technical solution of the present disclosure is achieved as follows.

A clamping method for clamping and compressing a target object comprises the steps of:
S10, fixing and placing a clamping apparatus in an experimental animal body by an operation, wherein the clamping apparatus comprises a magnet, a locking assembly and a clamping mechanism which is disposed outside the target object;
S20, applying an external magnetic field, unlocking the clamping mechanism by the locking assembly in response to an applied external magnetic field, and meanwhile providing an action force by the magnet in response to the applied external magnetic field so as to drive the clamping mechanism to clamp or release the target object; and S30, removing the external magnetic field, and locking the clamping mechanism by the locking assembly.

In some preferred embodiments, the clamping apparatus is a reversible self-locking clamping apparatus as described above.

In the clamping method provided by the present disclosure, by controlling the rotation direction of the applied external magnetic field, the clamping mechanism may be made to clamp or release the target object, thus achieving reversible clamping and compression on the target object.

### Brief Description of the Drawings

Fig. 1 is a schematic view of a reversible self-locking clamping apparatus in an exemplary embodiment of the present disclosure;
Fig. 2 is a front view of the reversible self-locking clamping apparatus shown in Fig. 1;
Fig. 3 is a sectional view taken along the direction A-A in Fig. 2;
Fig. 4 is a sectional view taken along the direction B-B in Fig. 2;
Fig. 5 is an enlarged view at C of Fig. 4, showing a structural schematic view in which a driving locking mechanism is in an unlocked state;
Fig. 6 is an enlarged view at C of Fig. 4, showing a structural schematic view in which the driving locking mechanism is in a locked state; and
Fig. 7 is a block flow diagram of a clamping method in another exemplary embodiment of the present disclosure;

In the drawings, 1, fixing support; 2, rotating mechanism; 3, driving locking mechanism; 4, sealing glue; 5, housing; 6, target object; 7, locking strap; 11, leg; 12, pinhole; 13, lock hole; 14, rotary shaft; 21, turntable; 22, rotary drum; 23, inner through-hole; 24, outer through-hole; 31, magnet; 32, reset element; 33, iron core; 34, lock head; 51, slit.

### Detailed Description of the Invention

The purpose, aspects, and advantages of the present disclosure will become more apparent from the detailed description set forth below when taken in conjunction with the drawings. It should be understood that the description is intended for purposes of illustration only, and is not intended to limit the scope of the present disclosure. Further, in the following description, descriptions of well-known structures and techniques are omitted to avoid unnecessarily obscuring the concepts of the present disclosure.

Various layer structure diagrams according to embodiments of the present disclosure are shown in the accompanying drawings. The figures are not drawn to scale, with some details exaggerated and possibly omitted for clarity. The shapes of the various regions and layers, and the relative sizes and positional relationships between them shown in the figures are merely exemplary, and in practice may deviate due to manufacturing tolerances or technical limitations. A person skilled in the art may additionally design regions/layers having different shapes, sizes, relative positions according to actual needs.

Obviously, the described embodiments are some, but not all, embodiments of the present application. Based on the embodiments in the present disclosure, all other embodiments obtained by a person skilled in the art without involving any inventive effort are within the scope of protection of the present disclosure.

In the description of the present disclosure, it should be noted that, the terms "first", "second" and the like are used solely for descriptive purposes and are not to be construed as indicating or implying relative importance.

Furthermore, the technical features involved in the various embodiments of the present disclosure described below may be combined with each other as long as they do not conflict with each other.

The embodiments of the present disclosure will now be described in detail by way of specific embodiments and their application with reference to the accompanying drawings.

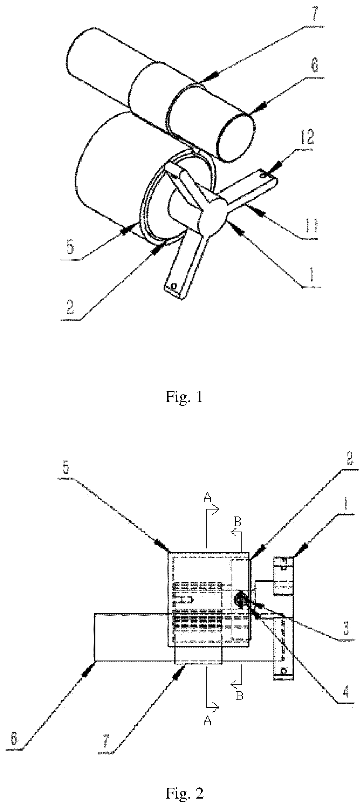

Referring to Figs. 1 and 2, the reversible self-locking clamping apparatus of the basic embodiment provided by the present disclosure includes a fixing support 1 for fixing the reversible self-locking clamping apparatus inside the body of an experimental animal, a rotating mechanism 2 rotatably mounted on the fixing support 1, a driving locking mechanism 3, and a clamping mechanism drivingly connected to the rotating mechanism 2.

Here, the fixing support 1 may be fixed inside the body of an experimental animal by any suitable means known in the art. In order to facilitate the fixing of the fixing support 1, the fixing support 1 includes legs 11. The number of legs 11 may be one or more. When there are a plurality of legs 11, the plurality of legs 11 are radially distributed around an axis, for example an embodiment having three legs 11 is shown in Fig. 1. The leg 11 is preferably provided with a pinhole 12 for suture-fixing the leg 11 in the animal body, so that the surgical suture may be firmly fixed on the leg 11 when the leg 11 is fixed in the animal body by the surgical suture. The number of the pinholes 12 provided in each leg 11 may also be one or more.

The fixing support 1 is provided with a part for mounting the rotating mechanism 2. Preferably, the fixing support 1 includes a rotary shaft 14. The rotating mechanism 2 is rotatably mounted on the rotary shaft 14. A bearing or the like may be disposed between the rotating mechanism 2 and the rotary shaft 14. The rotary shaft 14 is fixedly connected to the leg 11, i.e., the rotary shaft 14 is stationary relative to the leg 11. When the number of the legs 11 is plural, the plurality of legs 11 are uniformly distributed around the axis of the rotary shaft 14.

The driving locking mechanism 3 includes a magnet 31 and a locking assembly. The magnet 31 is capable of providing an action force to drive the rotation of the rotating mechanism 2 in response to an applied external rotating magnetic field. Herein, the magnet 31 is a magnetized permanent magnet, preferably a cylindrical permanent magnet. When the external magnetic field rotates, the magnet 31 generates a magnetically induced force, and the direction thereof is the direction of the magnetic line of maximum force pointing to the external magnetic field, i.e., generating a force to drive the magnetic pole direction of the magnet 31 to coincide with the external magnetic field, thereby driving the rotating mechanism 2 to rotate together with the rotation of the external magnetic field.

The locking assembly is capable of unlocking the rotating mechanism 2 in response to the applied external rotating magnetic field. The locking assembly locks the rotating mechanism 2 after the external rotating magnetic field is removed so that the rotating mechanism 2 cannot rotate. Namely, when the external magnetic field is applied, the locking assembly unlocks the rotating mechanism 2 under the action of the external magnetic field. At this time, the rotating mechanism 2 can rotate by the driving of the magnet 31. When the applied external magnetic field is removed, the locking assembly locks the rotating mechanism 2 and the fixing support 1 together, and the rotating mechanism 2 cannot rotate, thereby preventing the clamping mechanism from being loosened.

The clamping mechanism is capable of clamping and compressing or releasing a target object 6 that needs to be clamped in response to rotation of the rotating mechanism 2. Herein, the clamping mechanism is drivingly connected to the rotating mechanism 2, which means that when the rotating mechanism 2 rotates, the clamping mechanism can be driven to clamp or release the target object 6. When the rotating mechanism 2 does not rotate, the clamping mechanism is kept stationary synchronously, i.e., maintaining a clamping compression state on the target object 6. When the reversible self-locking clamping apparatus provided on the basis of the above-mentioned embodiment is used, the reversible self-locking clamping apparatus is firstly mounted in an experimental animal body by an operation, and then the locking assembly responds to the external rotating magnetic field by applying a positive external rotating magnetic field to the outside of the animal body to unlock the rotating mechanism 2. At this time, the rotating mechanism 2 can rotate on the fixing support 1. Also, the magnet 31 will generate a magnetically induced force under the action of the external rotating magnetic field, and the direction thereof is the direction of the maximum magnetic field line pointing to the external magnetic field, i.e., generating a force to drive the magnetic pole direction of the magnet 31 to coincide with the external magnetic field. Thus, the rotating mechanism 2 is driven to rotate, and the rotating mechanism 2 drives the clamping mechanism to apply clamping and compression to the target object (such as vascular or neural pressure) while rotating. Since the rotating mechanism 2 rotates with the rotation of the external magnetic field, the amount of compression applied by the clamping mechanism to the target object 6 may be precisely controlled by controlling the rotation angle and the number of turns of the external magnetic field, ensuring the control accuracy of the amount of compression.

When the external rotating magnetic field is removed, i.e., there is no external magnetic field, the locking assembly locks the rotating mechanism 2 so that the rotating mechanism 2 does not rotate any more. Since the clamping mechanism is drivingly connected to the rotating mechanism 2, the clamping mechanism also remains stationary, and a specific amount of compression may be continuously applied to the target object 6. When it is necessary to change the amount of compression on the target object, it is only necessary to apply the rotating magnetic field outside the animal body again. The compression force applied on the target object by the clamping mechanism may be accurately adjusted by controlling the rotation angle and the number of turns of the rotating magnetic field. When it is desired to reduce the amount of stress on the target object, it is only necessary to apply a counter-rotating magnetic field.

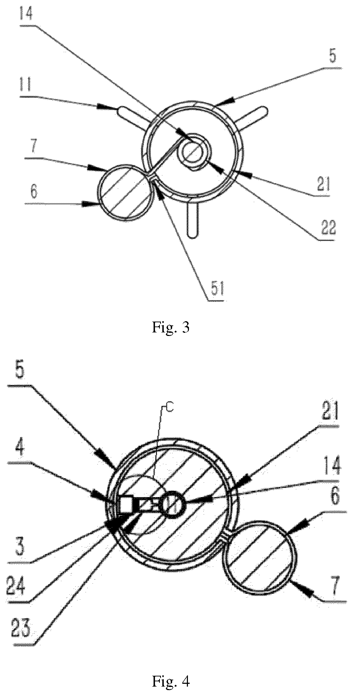

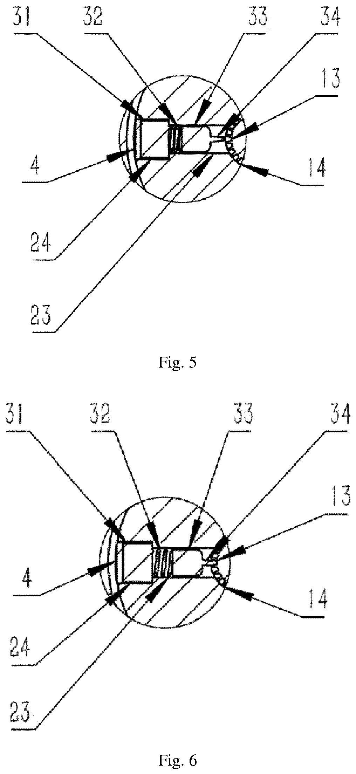

In the present disclosure, the locking assembly may employ a variety of existing locking apparatuses that can be non-contact controlled. In a preferred embodiment of the present disclosure, shown in Figs. 4 and 5, the locking assembly includes an iron core 33, a reset element 32, a lock head 34, and a lock hole 13. Here, the lock head 34 may be inserted into the lock hole 13 to perform a locking function. When the lock head 34 is separated from the lock hole 13, an unlocking function is performed. The lock head 34 is driven by the iron core 33. The lock head 34 can be drivingly connected to the iron core 33, or the lock head 34 may be fixed to the iron core 33. Alternatively, as shown in Fig. 5, the iron core 33 and the lock head 34 may be of an integral structure. The iron core 33 generates a magnetically induced force under the influence of the external magnetic field. The magnitude and direction of the magnetically induced force are determined by the applied magnetic field. Therefore, when the magnetically induced force generated on the iron core 33 by the external magnetic field is greater than the action force applied to the iron core 33 by the reset element 32, the iron core 33 drives the lock head 34 to leave the lock hole 13, namely, the iron core 33 drives the lock head 34 to leave the lock hole 13 under the action of the external rotating magnetic field so as to unlock the rotating mechanism 2. Referring to Fig. 6, when the external magnetic field is removed, the magnetic field strength on the iron core 33 decreases, and the reset element 32 drives the iron core 33 to be inserted into the lock hole 13 to lock the rotating mechanism 2. Therefore, the iron core 33 can respond to the change of the magnetic field to drive the lock head 34 to perform the self-locking and unlocking functions.

The position and the number of the lock holes 13 may be set as required, so long as the lock head 34 can be inserted into the lock hole 13 when the rotating mechanism 2 is rotated to a set angle. In order to improve the operability of the reversible self-locking clamping apparatus, preferably, as shown with reference to Figs. 5 and 6, the lock holes 13 are uniformly distributed around the rotary shaft of the rotating mechanism 2, with contact between adjacent lock holes 13. The lock hole 13 is counterbore.

Further preferably, the lock hole 13 is a tapered counterbore, i.e., the outer part (upper end) of the lock hole 13 is greater than the inner part (bottom). By providing the lock hole 13 as a tapered counterbore, it is possible to avoid that the lock head 34 cannot be inserted into the lock hole 13 when the actual rotation angle of the rotating mechanism 2 deviates from the set angle.

In the present disclosure, the reset element 32 may employ various components capable of providing an action force (e.g. spring force, magnetic force, etc.) for driving the lock head 34 towards the lock hole 13. Preferably, the reset element 32 is a spring that adjusts the sensitivity of the iron core 33 to changes in the external magnetic field. The reset element 32 shown in Fig. 5 is a compression spring.

In the present disclosure, the rotating mechanism 2 may adopt various structures capable of rotating on the fixing support 1. In a preferred embodiment, as shown in Fig. 3, the rotating mechanism 2 includes a turntable 21 which is rotatably mounted on the rotary shaft 14. The magnet 31 is mounted on the turntable 21 and located at an outer part of the turntable 21. The outer side of the turntable 21 refers to a side of the turntable 21 that is further away from the rotational axis of the turntable 21. In the present embodiment, by providing the rotating mechanism 2 as the turntable 21 and mounting the magnet 31 on the outer part of the turntable 21, the magnet 31 may have a larger distance from the rotational bearing, and a larger torque can be generated under the same driving force, thereby reducing the requirement for the magnetic field strength of the external rotating magnetic field.

As shown in Figs. 5 and 6, in a specific embodiment of the present disclosure, an outer through-hole 24 and an inner through-hole 23 are formed on the turntable 21. The outer through-hole 24 and the inner through-hole 23 are coaxially provided. Here, the inner through-hole 23 is located at an inner side of the turntable 21, has a slightly larger diameter than that of the iron core 33 and a smaller diameter than that of the magnet 31, which can provide guidance for the iron core 33 and the lock head 34. The outer through-hole 24 is located outside the turntable 21 and has a slightly larger diameter than that of the magnet 31 for mounting the magnet 31. A sealing glue 4 may be mounted at an outer end of the outer through-hole 24 to fix the magnet 31 to the turntable 21 so as to prevent the magnet 31 from falling off when the external magnetic field changes. Of course, the outer end of the outer through-hole 24 may be plugged in other ways, such as a bolt plug or the like.

The iron core 33 is slidably mounted in the inner through-hole 23. The lock head 34 is fixed on the iron core 33. The reset element 32 is disposed between the iron core 33 and the magnet 31. The lock holes 13 are formed on the rotary shaft 14. The lock holes 13 are uniformly distributed around the axis of the rotary shaft 14.

On the basis of the above-mentioned embodiment, it is further preferable that, as shown in Figs. 3 and 4, the rotating mechanism 2 further includes a drum 22 rotatably nested on the rotary shaft 14. The rotary shaft 14 is fixedly connected to and coaxially provided with the turntable 21. The outer diameter of the rotary drum 22 is less than the outer diameter of the turntable 21. The rotary drum 22 may have a gap between the rotary shaft 14 or be connected by a bearing component. In this embodiment, by providing the rotary drum 22 such that the rotary drum 22 has a smaller outer diameter, the magnet 31 may drive the rotary drum 22 to rotate with less force as the rotary drum 22 drives the clamping mechanism.

In the present disclosure, the clamping mechanism is used to convert rotational motion into clamping compression or release for the target object. Different types of clamping mechanisms may be used according to actual experimental requirements.

In the present disclosure, preferably, as shown in Figs. 1 and 3, the clamping mechanism includes a locking strap 7 capable of being wrapped outside the target object 6. One end of the locking strap 7 may be connected to the fixing support 1, and the other end of the locking strap 7 is connected to the rotating mechanism 2. When the locking strap 7 is tightened, the constriction can be formed for the target object 6. The locking strap 7 is preferably made of a biocompatible resin, so that the locking strap 7 has a good extensibility and a certain elasticity, is elastically deformed under the action of an external force, and returns to the original shape after the external force is removed, thereby enabling the clamping mechanism to better release the compression force on the blood vessel or nerve after reverse rotation.

In order to reduce the effect of the rotation of the rotating mechanism 2 on the tissue in the animal body, it is preferred that the reversible self-locking clamping apparatus further includes a housing 5, which is fixed to the fixing support 1. In particular, the housing 5 may be connected to the fixing support 1 by means of glue. The rotating mechanism 2 is disposed within the housing 5. Preferably, as shown in Fig. 3, the housing 5 is provided with a slit 51, and one end of the locking strap 7 is connected to the inside of the housing 5 or the edge of the slit 51. The other end of the locking strap 7 passes through the slit 51 and is connected to the rotating mechanism 2, i.e., the other end of the locking strap 7 is connected to the rotating mechanism 2 inside the housing 5 after passing through the slit 51 from the outside of the housing 5.

For a better understanding of the present disclosure, a preferred embodiment of the present disclosure is described in detail below.

As shown in Fig. 1, the embodiment of the present disclosure provides a reversible self-locking clamping apparatus controlled by a rotating magnetic field, including a fixing support 1, a rotating mechanism 2, a housing 5, a driving locking mechanism 3 and a clamping mechanism. The fixing support 1 includes a leg 11 and a rotary shaft 14 fixedly connected to the leg 11. A pinhole 12 is provided on the leg 11, and the leg 11 can be fixed in the animal body by using a surgical suture. A rotating mechanism 2 is mounted on the outside of the rotary shaft 14, and the rotating mechanism 2 may rotate on the rotary shaft 14.

The rotating mechanism 2 includes a turntable 21 and a drum 22. The turntable 21 and the rotary drum 22 are rotatably mounted on the rotary shaft 14. the rotary drum 22 is provided coaxially with the turntable 21 and fixedly connected to the turntable 21 so that the turntable 21 may rotate with the rotating magnetic field.

As shown in Figs. 3-5, the turntable 21 is provided with an inner through-hole 23 and an outer through-hole 24 for the driving locking mechanism 3. The driving locking mechanism 3 includes a magnet 31 and a locking assembly which includes a reset element 32 such as a spring, an iron core 33, a lock head 34 and a lock hole 13.

Here, the magnet 31 is fixed in the outer through-hole 24 of the turntable 21 via the sealing glue 4, and is connected to the iron core 33 via a spring. The rotary shaft 14 is provided with lock holes 13 arranged in a uniform annular manner. The top end of the iron core 33 is connected to the lock head 34. When the iron core 33 is acted by the superimposed magnetic field of the magnet 31 and the external magnetic field, a magnetically induced force is generated, and the magnitude and direction of the magnetically induced force are determined by the superimposed magnetic field. As shown in Fig. 5, when the direction of the external magnetic field is consistent with the direction of the magnetic field of the magnet 31, the magnetically induced force of the iron core 33 is the maximum, and the spring is compressed, so that the lock head 34 leaves the lock hole 13, thereby allowing the turntable 21 to rotate. When the external magnetic field rotates, the magnet 31 will generate a magnetically induced force, and the direction thereof is the direction of the magnetic line of maximum force pointing to the external magnetic field, namely, generating a force to drive the magnetic pole direction of the magnet 31 to coincide with the external magnetic field, and then driving the turntable 21 to rotate together with the rotation of the external magnetic field. As shown in Fig. 6, when the external magnetic field is removed, the magnetic field strength on the iron core 33 decreases, and the magnetically induced force thereof is less than the thrust force of the spring, and the spring pushes the iron core 33 away from the magnet 31. Thus, the lock head 34 is inserted into the lock hole 13 to form a self-locking, thereby locking the clamping mechanism.

The housing 5 is wrapped outside the rotating mechanism 2 for protecting the target object 6, such as a blood vessel or a nerve, from the possible presence of the rotating mechanism 2.

The clamping mechanism includes a locking strap 7. One end of the locking strap 7 is fixed on the inner side of the housing 5, and the other end is connected to the rotary drum 22 via a slit 51 on the housing 5 after being wrapped around the target object 6 such as a blood vessel or a nerve. When the turntable 21 and the rotary drum 22 rotate in a forward direction, the locking belt 7 is tightened so as to generate pressure on blood vessels or nerves. The degree of compression is determined by the rotation angle and number of turns of the turntable 21 and the rotary drum 22. When the turntable 21 and the rotary drum 22 are counter-rotated, the locking strap 7 is released, thereby reducing the compression of blood vessels or nerves, which thus can be used to simulate the relief of compression symptoms.

Based on the reversible self-locking clamping apparatus provided by the above embodiments, the present disclosure also provides a rotary self-locking mechanism that may be used to provide a driving force for performing clamping compression or the like in the animal body. Referring to Fig. 4, the rotary self-locking mechanism includes a rotary shaft 14, a rotating mechanism 2 rotatably mounted on the rotary shaft 14, and a driving locking mechanism 3. The rotary shaft 14 is used to provide a supporting shaft for the rotation of the rotating mechanism 2, and may be fixed in the animal body by means of legs or the like.

With continued reference to Fig. 4, the rotating mechanism 2 may be mounted on the rotary shaft 14 via a bearing or the like for providing rotational movement. The driving locking mechanism 3 provides a force for driving the rotating mechanism 2 in response to an applied external rotating magnetic field, and provides the function of locking the position of the rotating mechanism 2 after the external rotating magnetic field is removed. The driving locking mechanism 3 includes a magnet 31 and a locking assembly. The magnet 31 is capable of providing an action force to drive the rotation of the rotating mechanism 2 in response to an applied external rotating magnetic field. The magnet 31 is a magnetized permanent magnet, preferably a cylindrical permanent magnet, mounted in the rotating mechanism 2 for generating a rotational force to drive the rotating mechanism 2 to rotate as the direction of the external magnetic field changes in response to the external magnetic field.

The locking assembly is capable of unlocking the rotating mechanism 2 in response to the applied external rotating magnetic field. The locking assembly locks the rotating mechanism 2 after the external rotating magnetic field is removed so that the rotating mechanism 2 cannot rotate.

Preferably, referring to Figs. 5 and 6, the locking assembly includes the locking assembly comprises an iron core 33, a reset element 32, a lock head 34 and a lock hole 13. The iron core 33 is capable of driving the lock head 34 away from the lock hole 13 under the action of an external rotating magnetic field to unlock the rotating mechanism 2. The reset element 32 drives the iron core 33 into the lock hole 13 to lock the rotating mechanism 2 when the external rotating magnetic field is removed. The iron core 33 may be mounted in the rotating mechanism 2, with one end connected to a reset element 32 (e.g., a spring) and the other end connected to a lock head 34 for responding to changes in magnetic field strength. With changes in the superposed magnetic field of the external magnetic field and the magnet 31, different amounts of magnetically induced force may be generated to move the lock head 34. A spring is interposed between the magnet 31 and the iron core 33 to generate a pushing force to push the iron core 33 away from the magnet 31. The lock holes 13 are preferably disposed on the rotary shaft 14 in the form of uniformly annular counterbores in order to provide a fixing force required for the driving locking mechanism 3 in a manner that it is a tapered counterbore with the outer part being larger than the inner part.

With continuing reference to Figs. 5 and 6, in the above-mentioned structure, when the external magnetic field is applied to a certain size and direction, the superposed magnetic field on the iron core 33 becomes larger, and the magnetically induced force is greater than the spring force. The spring is compressed by the iron core 33. The lock head 34 on the iron core 33 leaves the lock hole 13, allowing the rotating mechanism 2 to rotate freely. When the external magnetic field is removed, the iron core 33 is only attracted by the magnet 31, and the magnetically induced force thereof is less than the thrust force of the spring. The lock head 34 is pushed into the lock hole 13, forming a self-locking.

As shown in Fig. 3, the rotating mechanism 2 includes a turntable 21 rotatably mounted on the rotary shaft 14, and a magnet 31 mounted on the turntable 21 at an outer part of the turntable 21. The larger diameter of the turntable 21 allows a larger torque to be generated at the same driving force. The turntable 21 may be used for accommodating the driving locking mechanism 3. In particular, the turntable 21 is provided with an outer through-hole 24 and an inner through-hole 23, the outer through-hole 24 and the inner through-hole 23 being coaxially arranged.

The inner through-hole 23 is located at an inner position of the turntable 21 (the direction close to the rotational axis of the rotary disc being inner, and the direction away from the rotational axis of the rotary disc being outer), and has a diameter slightly larger than that of the iron core 33 and smaller than the magnet 31, which provides guidance for the iron core 33 and the lock head 34. The outer through-hole 24 is located outside the turntable 21 and is concentric with the inner through-hole 23 and has a slightly larger diameter than that of the magnet 31 for mounting the magnet 31. The magnet 31 is fixedly mounted in the outer through-hole 24, and the outer end opening of the outer through-hole 24 is closed off by the sealing glue 4.

The iron core 33 is slidably mounted in the inner through-hole 23. The lock head 34 is fixed on the iron core 33. The reset element 32 is disposed between the iron core 33 and the magnet 31. The lock holes 13 are formed on the rotary shaft 14. The lock holes 13 are uniformly distributed around the axis of the rotary shaft 14. The reset element 32 is located between the magnet 31 and the iron core 33, and generates a pushing force to push the iron core 33 away from the magnet 31. When an external magnetic field is applied to a certain size and direction, the superposed magnetic field on the iron core 33 becomes larger, and the magnetically induced force is greater than the reset force of the reset element 32, the reset element 32 is compressed by the iron core 33, and the lock head 34 on the iron core 33 leaves the lock hole 13, allowing the turntable 21 to rotate freely. When the external magnetic field is removed, the iron core 33 is only attracted by the magnet 31, and the magnetically induced force thereof is less than the reset force of the reset element 32. Thus, the lock head 34 will be pushed into the lock hole 13, forming a self-locking. The reset element 32 may be a spring.

A rotary drum 22 may be further mounted at one end of the turntable 21. The rotary drum 22 is mounted outside the rotary shaft 14, fixed to a side of the turntable 21, and rotates together with the turntable 21 to output a rotary motion.

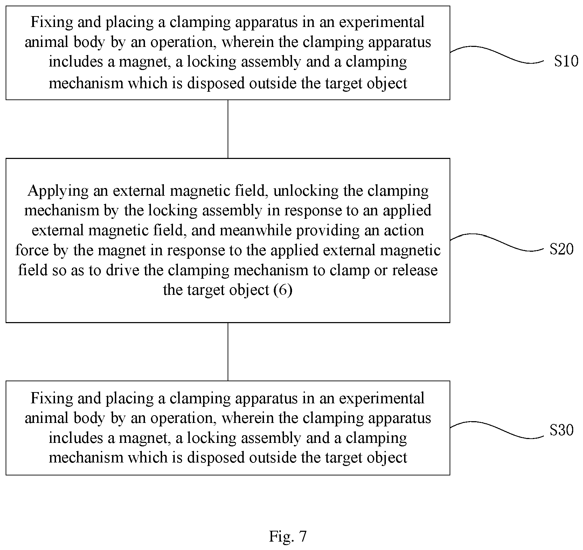

Meanwhile, as shown in Fig. 7, the present disclosure also provides a clamping method for clamping and compressing a target object in an experimental animal, including the steps of:
S10, fixing and placing a clamping apparatus in an experimental animal body by an operation, wherein the clamping apparatus includes a magnet, a locking assembly and a clamping mechanism which is disposed outside the target object;
S20, applying an external magnetic field, unlocking the clamping mechanism by the locking assembly in response to an applied external magnetic field, and meanwhile providing an action force by the magnet in response to the applied external magnetic field so as to drive the clamping mechanism to clamp or release the target object; and S30, removing the external magnetic field, and locking the clamping mechanism by the locking assembly.

Here, in step S20, different external magnetic fields may be set according to the type of clamping apparatus. Preferably, the external magnetic field is a rotating magnetic field. The clamping apparatus is a reversible self-locking clamping apparatus as described in the above-mentioned embodiments. By applying the external rotating magnetic field, its internal rotating parts may be driven in the forward or reverse direction to generate a controlled degree of compression and constriction or release on the external target object.

In summary, the above-mentioned technical solutions of the present disclosure have the following beneficial technical effects.

The reversible self-locking clamping apparatus and method provided by the present disclosure may be surgically implanted into a living experimental animal, and may wait until the experimental animal recovers after the operation to gradually clamp by non-contact application of the external rotating magnetic field, so that the experimental result would not be affected by stress stimulation such as the operation. After the external magnetic field is removed, the self-locking device inside the self-locking device will automatically lock the position, so that the clamping state is maintained in the absence of the external magnetic field, thereby enabling the long-term chronic constriction test on the experimental animal. By controlling the rotation angle and the number of turns of the external rotating magnetic field, the amount of compression of the clamp can be accurately controlled, thus ensuring the control accuracy of the compression, avoiding the error caused by the artificial ligation in the traditional surgical method, making the experimental results more stable and reliable. Due to the high repeatability and controllable degree and time of constriction, the number of experimental animals required can be greatly reduced, thereby improving the speed and efficiency of the experiment and greatly reducing the human resources and costs required for the experiment.

The order of the above-mentioned detailed description is merely for convenience of description and does not represent the advantages or disadvantages of the embodiments.

Finally, it should be noted that the above-mentioned detailed description are only intended to illustrate the technical solution of the present disclosure, but not to limit it. Although the present disclosure has been described in detail with reference to the foregoing detailed description, those skilled in the art will appreciate that the technical solutions of the above-mentioned detailed description can still be modified, or some of the technical features thereof can be equivalently substituted. However, with such modifications and substitutions, the essence of the corresponding technical solutions does not depart from the spirit and scope of the detailed description of the present disclosure.

## Claims

1. A reversible self-locking clamping apparatus, **characterized by** comprising a fixing support (1) configured for fixing the reversible self-locking clamping apparatus inside the body of an experimental animal, a rotating mechanism (2) rotatably mounted on the fixing support (1), a driving locking mechanism (3), and a clamping mechanism drivingly connected to the rotating mechanism (2), wherein the driving locking mechanism (3) comprises a magnet (31) and a locking assembly; the magnet (31) is capable of providing an action force for driving the rotation of the rotating mechanism (2) in response to an applied external rotating magnetic field; the locking assembly is capable of unlocking the rotating mechanism (2) in response to the applied external rotating magnetic field; the locking assembly locks the rotating mechanism (2) after the external rotating magnetic field is removed so that the rotating mechanism (2) cannot rotate; and the clamping mechanism is capable of clamping and compressing or releasing a target object (6) that needs to be clamped in response to the rotation of the rotating mechanism (2).

2. The reversible self-locking clamping apparatus according to claim 1, **characterized in that** the locking assembly comprises an iron core (33), a reset element (32), a lock head (34) and a lock hole (13), wherein the iron core (33) is capable of driving the lock head (34) away from the lock hole (13) under the action of an external rotating magnetic field to unlock the rotating mechanism (2); and the reset element (32) drives the iron core (33) into the lock hole (13) to lock the rotating mechanism (2) when the external rotating magnetic field is removed.

3. The reversible self-locking clamping apparatus according to claim 2, **characterized in that** the lock hole (13) is a tapered counterbore.

4. The reversible self-locking clamping apparatus according to claim 2, **characterized in that** the reset element (32) is a spring.

5. The reversible self-locking clamping apparatus according to claim 2, **characterized in that** the fixing support comprises a rotary shaft (14); the rotating mechanism (2) comprises a turntable (21) which is rotatably mounted on the rotary shaft (14); and the magnet (31) is mounted on the turntable (21) and located at an outer part of the turntable (21).

6. The reversible self-locking clamping apparatus according to claim 5, **characterized in that** an outer through-hole (24) and an inner through-hole (23) are disposed on the turntable (21); the outer through-hole (24) and the inner through-hole (23) are coaxially disposed; the magnet (31) is fixedly mounted in the outer through-hole (24); the iron core (33) is slidably mounted in the inner through-hole (23); the lock head (34) is fixed on the iron core (33); the reset element (32) is disposed between the iron core (33) and the magnet (31); the lock holes (13) are formed on the rotary shaft (14); and the lock holes (13) are uniformly distributed around the axis of the rotary shaft (14).

7. The reversible self-locking clamping apparatus according to claim 6, **characterized in that** the rotating mechanism (2) further comprises a rotary drum (22) rotatably nested on the rotary shaft (14); the rotary drum (22) is fixedly connected to and coaxially provided with the turntable (21); and the outer diameter of the rotary drum (22) is less than the outer diameter of the turntable (21).

8. The reversible self-locking clamping apparatus according to claim 1, **characterized in that** the reversible self-locking clamping apparatus further comprises a housing (5); the housing (5) is fixed to the fixing support (1); and the rotating mechanism (2) is disposed inside the housing (5).

9. The reversible self-locking clamping apparatus according to claim 8, **characterized in that** the clamping mechanism comprises a locking strap (7), which is capable of being wrapped outside the target object; the housing (5) is provided with a slit (51); one end of the locking strap (7) is connected to the inside of the housing (5) or the edge of the slit (51), and the other end of the locking strap (7) passes through the slit (51) and is connected to the rotating mechanism (2).

10. The reversible self-locking clamping apparatus according to claim 9, **characterized in that** the locking strap (7) is made of a biocompatible resin.

11. The reversible self-locking clamping apparatus according to claim 1, **characterized in that** the fixing support (1) comprises a leg (11) and a rotary shaft (14) fixedly mounted on the leg (11); and the leg (11) is provided with a pinhole (12) for suture-fixing the leg (11) in vivo of the animal.

12. A rotary self-locking mechanism, **characterized by** comprising a rotary shaft (14), a rotating mechanism (2) rotatably mounted on the rotary shaft (14), and a driving locking mechanism (3); the driving locking mechanism (3) comprises a magnet (31) and a locking assembly, wherein the magnet (31) is capable of providing an action force for driving the rotation of the rotating mechanism (2) in response to an applied external rotating magnetic field, and the locking assembly is capable of unlocking the rotating mechanism (2) in response to the applied external rotating magnetic field; the locking assembly locks the rotating mechanism (2) after the external rotating magnetic field is removed so that the rotating mechanism (2) cannot rotate.

13. The rotary self-locking mechanism according to claim 12, **characterized in that** the locking assembly comprises an iron core (33), a reset element (32), a lock head (34) and a lock hole (13), wherein the iron core (33) is capable of driving the lock head (34) away from the lock hole (13) under the action of an external rotating magnetic field to unlock the rotating mechanism (2); and the reset element (32) drives the iron core (33) into the lock hole (13) to lock the rotating mechanism (2) when the external rotating magnetic field is removed.

14. The rotary self-locking mechanism according to claim 13, **characterized in that** the lock hole (13) is a tapered counterbore; and the reset element (32) is a spring.

15. The rotary self-locking mechanism according to claim 13, **characterized in that** the rotating mechanism (2) comprises a turntable (21) which is rotatably mounted on the rotary shaft (14); and the magnet (31) is mounted on the turntable (21) and located at an outer part of the turntable (21).

16. The rotary self-locking mechanism according to claim 15, **characterized in that** an outer through-hole (24) and an inner through-hole (23) are disposed on the turntable (21); the outer through-hole (24) and the inner through-hole (23) are coaxially disposed; the magnet (31) is fixedly mounted in the outer through-hole (24); the iron core (33) is slidably mounted in the inner through-hole (23); the lock head (34) is fixed on the iron core (33); the reset element (32) is disposed between the iron core (33) and the magnet (31); the lock holes (13) are formed on the rotary shaft (14); and the lock holes (13) are uniformly distributed around the axis of the rotary shaft (14).

17. A clamping method for clamping and compressing a target object (6), **characterized by** comprising the steps of:
S10, fixing and placing a clamping apparatus in an experimental animal body by an operation, wherein the clamping apparatus comprises a magnet, a locking assembly and a clamping mechanism which is disposed outside the target object;
S20, applying an external magnetic field, unlocking the clamping mechanism by the locking assembly in response to an applied external magnetic field, and meanwhile providing an action force by the magnet in response to the applied external magnetic field so as to drive the clamping mechanism to clamp or release the target object (6); and
S30, removing the external magnetic field, and locking the clamping mechanism by the locking assembly.

18. The clamping method according to claim 17, **characterized in that** the clamping apparatus is the reversible self-locking clamping apparatus according to claim 1.
